Europäisches Patentamt

European Patent Office

Office européen des brevets

(19)

(11) Publication number : **0 404 470 B1**

## (12) EUROPEAN PATENT SPECIFICATION

(45) Date of publication of patent specification :
13.04.94 Bulletin 94/15

(51) Int. Cl.⁵ : **A61K 7/46, C11D 3/50**

(21) Application number : **90306599.3**

(22) Date of filing : **18.06.90**

(54) **Fragrance compositions and their use in detergent products.**

The file contains technical information
submitted after the application was filed and
not included in this specification

(30) Priority : **19.06.89 GB 8914055**

(43) Date of publication of application :
**27.12.90 Bulletin 90/52**

(45) Publication of the grant of the patent :
**13.04.94 Bulletin 94/15**

(84) Designated Contracting States :
**CH DE ES FR GB IT LI NL SE**

(56) References cited :
**EP-A- 0 147 191**

(56) References cited :
**DE-C- 2 919 513**
**US-A- 4 288 341**
**US-A- 4 322 308**
**US-A- 4 343 783**

(73) Proprietor : **QUEST INTERNATIONAL B.V.**
**Huizerstraatweg 28**
**NL-1411 GP Naarden (NL)**

(72) Inventor : **Behan, John Martin**
**Shermel, Ball Lane**
**Kennington, Ashford, Kent (GB)**
Inventor : **Clements, Christopher Francis**
**102 Bouverie Road West**
**Folkestone Kent CT20 2LB (GB)**

(74) Representative : **Ford, Michael Frederick et al**
**MEWBURN ELLIS 2 Cursitor Street**
**London EC4A 1BQ (GB)**

## Description

This invention relates to compositions of fragrance materials and their use in detergent products.

In particular embodiments, the invention relates to combinations of such fragrance compositions with detergent powders, e.g. granulates, with liquids, especially for example those intended for fabric-washing, with soap and detergent bars and pastes, with fabric-conditioning compositions e.g. in liquid or solid (sheet) form, and with personal body deodorant compositions, e.g. underarm deodorants.

### Prior Art

EP-A-0 003 171, EP-A-0 003 172 and DE-C-2919513 (Unilever) and US-A-4 322 308, US-A-4 343 783, US-A-4 288 341 and US-A-4 304 679 (Lever) (and the references cited therein) give extensive known examples of perfumery compositions especially with reference to their use in detergents.

EP 0 003 171 and EP 003 172 also disclose compositions in which the perfume compositions have a deodorant effect when they are used.

Further examples of perfumery compositions, as well as their application to enzyme-containing detergents, are given in JP 57-85898 and JP 57-85900 (Lion Corp), and in USP 4 515 705 (Procter & Gamble).

EP 0 147 191 (Unilever) gives further examples of perfumery compositions, having not only deodorant effect but also stability in the presence of bleaching compositions.

It is also proposed in GB 1 589 866 to incorporate 0.3-3% of ester of citrate or acetylcitrate (eg. triethyl citrate) as a deodorant in a soap bar.

### Summary of the Present invention

It has surprisingly been found that certain compositions of fragrance materials can confer deodorant effects in use even though they have in themselves a low or imperceptible level of fragrance (low odour intensity). This is of advantage in many applications where an intense fragrance is not desired, while a deodorant effect is to be welcomed.

According to an aspect of the invention, there is provided a composition of fragrance materials having

(a) an odour Intensity less than that of a 10% solution of benzyl acetate in dipropylene glycol (which corresponds to an Odour Intensity Index of less than about 100, when tested according to an Odour Intensity Test as described below), and

(b) a Malodour Reduction Value of at least about 0.25, preferably, at least about 0.5, when tested according to the test procedure set out in European Patent Application No. 0 147 191 or an Odour Reduction Value of about 0.25, preferably at least about 0.5, when tested according to the test procedure set out in European Patent Application No. 0 003 172.

It is understood that in this specification, expressions such as 'perfume' and 'fragrance' extend to compositions of which the odour intensity may be so low as to be imperceptible in use.

A suitable selection of examples of perfumery materials for incorporation into such compositions is for example provided in the Examples below. More generally, any of a wide range of perfumery materials may be incorporated into the compositions, provided that the basis of selection is such as to provide a deodorant effect, and the odour intensity index of the resulting composition is as defined above.

Extensive directions for the selection of materials in order to provide a deodorant effect are given for example in EP 0 147 191 EP 0 003 172, and USP 4 304 679.

It is helpful if the bulk of the individual ingredients chosen for the composition also individually possess an Odour Intensity Index less than about 110, preferably less than about 100, or even lower. Small quantities of more intense materials may however be tolerated, e.g. for the purpose of adjusting the mild perfume note which may be given by the overall composition.

In a number of particular embodiments,the compositions can comprise at least 30% by weight of a musk, e.g. at least 35% or at least 40%. Where musks are present, either in these or in other amounts, they can usefully be selected from musks such as galaxolide (TM) (IFF) (in class 3 defined below) and/or Traseolide (TM) (Quest) (in class 4 defined below).

According to another aspect of the invention, there is provided a detergent composition suitable for fabric-washing, comprising perfume ingredients containing

(a) optionally at least about 0.03% of a musk, based on the weight of the detergent composition,

(b) at least about 0.07% of other perfume components,

provided that the fragrance components (a) and (b) together constitute a composition as defined above, so that it has an odour intensity less than that of a 10% solution of benzyl acetate in dipropylene glycol

and the aggregate of fragrance components together pass either of the odour reduction tests cited above.

The compositions of fragrance materials with low odour intensity may usefully be incorporated, according to this invention, in various detergent and personal care products in for example the following amounts (but without limitation):- usually about 0.1% or more; in fabric-washing compositions about 0.1-0.3%; in concentrated fabric-washing compositions up to about 0.8%: in fabric-conditioning liquids up to about 0.3%; in sheet-form fabric-conditioning solid preparations up to about 5%; in soap and detergent bars and pastes about 0.2-1.8%.

In certain embodiments, the invention also provides a deodorant perfume which comprises deodorant perfume components, eg. those which are judged to be stable in the presence of sodium perborate tetrahydrate and N,N,N'N'-tetraacetyl ethylenediamine (TAED) according to the Bleach Stability Test, as described in EP 0 147 191, the deodorant perfume having a Malodour Reduction Value of from 0.25 to 3.0 as measured by the Malodour Reduction Value Test, and also having the characteristic low odour intensity defined elsewhere herein.

Such deodorant perfumes can comprise from 50 to 100% by weight of (eg. bleach-stable) deodorant perfume components and from 0 to 50% by weight of further ingredients, said deodorant perfume components for example having a Lipoxidase-Inhibiting Capacity of at least 50% or a Raoult Variance Ratio of at least 1.1, and (where bleach stability is also desired) also being judged to be stable in the presence of sodium perborate tetrahydrate and N,N,N'N'-tetraacetyl ethylenediamine (TAED) according to the Bleach Stability Test, said components being allocated to one of six classes consisting of:

Class 1 :       Phenolic substances
Class 2 :       Essential oils, extracts, resins and synthetic oils (denoted "AB")
Class 3 :       Aldehydes and ketones
Class 4 :       Nitrogen-containing compounds and polycyclic compounds
Class 5 :       Esters
Class 6 :       Alcohols and ethers

provided that where a deodorant perfume component can be assigned to more than one class, it is allocated to the class having the lower or lowest number; said components being so selected that:

a) the deodorant perfume contains at least five different components preferably at least one from each of class 1, 2 and 4;

b) the deodorant perfume contains components from at least four of the six classes; and

c) any component present in the deodorant perfume at a concentration of less than 0.5% by weight of the said perfume is eliminated from the requirements of (a) and (b),

said deodorant perfume having a Malodour Reduction Value of from 0.25 to 3.0 as measured by the Malodour Reduction Value Test which comprises the steps of:

i) selecting pieces of 100% bulked polyester sheet shirt fabric of 20cm x 20cm;

ii) washing the selected pieces of fabric in a front-loading drum-type washing machine with a standard unperfumed washing powder containing the following ingredients:

|  | Parts by weight |
|---|---|
| Sodium dodecylbenzene sulphonate | 9 |
| $C_{13-15}$ alcohol 7EO | 4 |
| Sodium tripolyphosphate | 33 |
| Alkaline sodium silicate | 6 |
| Sodium carboxymethyl cellulose | 1 |
| Magnesium silicate | 1 |
| Ethylenediamine tetraacetic acid | 0.2 |
| Sodium sulphate | 15 |
| Water | 10.8 |

iii) rinsing the washed pieces of fabric and drying them to provide "untreated" fabric;

iv) re-washing half of the "untreated" pieces of fabric in the washing machine with the standard washing powder to which has been added 0.2% by weight of a bleach-stable perfume under test, rinsing and re-drying to provide "treated" pieces of fabric;

v) inserting the "treated" and "untreated" pieces of fabric into clean polyester cotton shirts in the underarm region so that in each shirt, one underarm region receives a "treated" fabric insert and the other underarm region receives an "untreated" fabric insert in accordance with a statistical design;

vi) placing the shirts carrying the inserts on a panel of 40 Caucasian male subjects of age within the range of from 20 to 55 years (the subjects being chosen from those who develop axillary body malodour that is not unusually strong and who do not develop a stronger body malodour in one axilla compared with the other);

vii) assessing the body malodour of the fabric inserts after a period of five hours whereby three trained female assessors record the olfactory intensity of malodour on a 0 to 5 scale, 0 representing no odour and 5 representing very strong malodour, the strength of the odour in each instance being related for purposes of comparison to standard odours produced by aqueous solutions of isovaleric acid at different concentrations according to the following table:

| Score | Odour level | Conc. of aqueous isovaleric acid (ml/l) |
|-------|-------------|------------------------------------------|
| 0 | No odour | 0 |
| 1 | Slight | 0.013 |
| 2 | Definite | 0.053 |
| 3 | Moderate | 0.22 |
| 4 | Strong | 0.87 |
| 5 | Very Strong | 3.57 |

viii) calculating the average scores for both treated fabric and untreated fabric, and subtracting the average score of the treated fabric from the average score of the untreated fabric to arrive at the Malodour Reduction Value for the bleach-stable perfume, the bleach-stable perfume being designated a bleach-stable deodorant perfume when its Malodour Reduction Value is from 0.25 to 3.0;

the Bleach Stability Test comprising the steps of:

i) dosing a perfume material into the standard unperfumed washing powder and incubating the dosed powder at 20°C in a sealed container for seven days;

ii) dividing the dosed powder into two portions and adding to each portion sodium perborate tetrahydrate together with either TAED granules or sodium sulphate (to act as an inert filler in place of TAED) to provide test and control formulations having the following constitution:

| | % w/w | |
|---|---|---|
| | Test Powder | Control Powder |
| Standard unperfumed powder | 76 | 76 |
| Perfume material under test | 0.2 | 0.2 |
| Sodium perborate tetrahydrate | 13 | 13 |
| TAED granules (65% TAED) | 10.8 | - |
| Sodium sulphate | - | 10.8 |

4

iii) incubating both test and control powders in sealed containers at 45°C for a further seven days; and

iv) assessing samples of the test and control powders according to a standard triangle test as described in "Manual on Sensory Testing Methods" published by the American Society for Testing and Materials (1969), using a panel of 20 assessors, who are instructed to judge by smell which of the three powder samples is the odd one out, the perfume material being designated a bleach-stable deodorant perfume component when the odd one out is correctly identified by no more than 9 of the 20 assessors.

Deodorant perfume components can be classified into six chemically defined classes. The perfume components may be described in terms of four categories, each of which is given below together with examples of components which are to be assigned to each category.

1) Single chemical compounds whether natural or synthetic, for example, hexyl salicylate: the majority of components are in this category.

2) Synthetic reaction products (products of reaction), mixtures of isomers and possibly homologues, for example, alpha-iso-methyl ionone.

3) Natural oils and extracts, for example, benzoin Siam resinoid.

4) Synthetic oils (eg. analogues of category 3): this category includes materials that are not strict analogues of natural oils but are materials that result from attempts to copy or improve upon certain natural oils, for example Bergamot AB 430.

Components of Categories (3) and (4) although often uncharacterised chemically are available commercially.

Where a material is supplied or used conventionally for convenience as a mixture, e.g. p-t-amyl cyclohexanone diluted with diethyl phthalate, for the purposes of this specification two components are present, so that use of 5% of a blend of 1 part of this ketone and 9 parts of diethylphthalate is represented as 0.5% of the ketone and 4.5% of diethyl phthalate.

It has been found advantageous in formulating the most effective deodorant perfumes to use components that, as well as satisfying the lipoxidase or morpholine tests satisfy further conditions. These conditions are:

i) there are at least five different components present conforming with the classification below;

ii) there are represented components from at least four different chemical classes (defined below);

iii) at least 50%, preferably at least 55% and most preferably from 60 to 100% by weight of the deodorant perfumes comprise deodorant perfume components conforming with the classification below;

iv) a component is not considered to contribute to the efficacy of the bleach-stable deodorant perfume if it is present in that perfume at a concentration of less than 0.5% by weight.

Each component should be allocated to one of six classes. These classes are: Class 1 - Phenolic substances;

2 - Essential oils, extracts, resins and synthetic oils (denoted "AB");

3 - Aldehyde and ketones;

4 - Nitrogen-containing compounds;

5 - Esters;

6 - Alcohols and ethers.

In assigning a component to a class, the following rules are to be observed. Where the component could be assigned to more than one class, the component is allocated to the class occurring first in the order given above: for example methyl anthranilate, which is a nitrogen-containing compound, is placed in Class 4, although as an ester it otherwise might have been allocated to Class 5. Similarly, ethyl salicylate, which is phenolic in character, is allocated to Class 1 instead of Class 5.

The following are examples of (also bleach-stable) deodorant perfume components that have either a Lipoxidase Inhibiting Capacity (LIC value) of at least 50% or a Raoult Variance Ratio (RVR value) of at least 1.1, and additionally have a Bleach Stability Test (BST) panel score of up to 9, indicating that they are judged to be bleach-stable. Their class, molecular weight (m), LIC and RVR values and BST panel scores as determined by the tests already described herein are also indicated.

The nomenclature adopted for the components listed below and for the perfume ingredients which appear in the perfume formulations of Examples 1 to 7 is, so far as is possible, that employed by Steffen Arctander in "Perfume and Flavour Chemicals (Aroma Chemicals)" Volume I and II (1969) and the "Perfume and Flavour Chemicals (Aroma Chemicals)" Volume I and II (1969) and the "Perfume & Flavour Materials of Natural Origin" (1960) by the same author. Where a component or ingredient is not described by Arctander, then either the chemical name is given or, where this is not known the perfumery house speciality code name is given. Note that synthetic oils denoted "AB" are available from Quest International Limited.

Specific examples of perfume components are:

Class 1 - Phenolic Substances

| | LIC value | RVR value | m | BST panel score |
|---|---|---|---|---|
| iso-Amyl salicylate | 95 | 1.24 | 208 | 9 |
| Carvacrol | 32 | 1.43 | 150 | 6 |
| Clove leaf oil | 79 | 1.43 | 164 | 5 |
| Ethyl salicylate | - | 1.19 | 194 | 7 |
| iso-Eugenol | 100 | 1.48 | 164 | 4 |
| Hexyl salicylate | 100 | - | 222 | 5 |
| Thyme oil red | 55 | 1.37 | 150 | 9 |

Class 2 - Essential oils, extracts, resins and synthetic oils (denoted "AB")

| | | | | |
|---|---|---|---|---|
| Bergamot AB 430 | 58 | 0.97 | 175 | 7 |
| Geranium AB 76 | 26 | 1.29 | 154 | 6 |
| Rose AB 380 | 0 | 1.28 | 175 | 9 |
| Rose AB 409 | 35 | 1.34 | 175 | 8 |

Class 3 - Aldehydes and ketones

| | | | | |
|---|---|---|---|---|
| 7-Acetyl-1,1,3,4,4,6-hexamethyl-tetrahydro-naphthalene | 100 | 1.03 | 258 | 8 |
| p-t-Amyl cyclohexanone | 50 | 1.10 | 182 | 8 |
| 2-n-Heptylcyclo-pentanone | 56 | 1.05 | 182 | 7 |
| α-iso-Methyl ionone | 100 | 1.13 | 206 | 7 |
| β-Methyl naphthyl ketone | 100 | 0.96 | 170 | 3 |

Class 4 - Nitrogen-containing compounds

| | LIC value | RVR value | m | BST panel score |
|---|---|---|---|---|
| iso-Butyl quinoline | - | 1.10 | 185 | 5 |
| Methyl anthranilate | 69 | 1.20 | 151 | 6 |

6

Class 5 - Esters

| o-t-Butylcyclohexyl | | | | |
|---|---|---|---|---|
| acetate | 52 | 1.08 | 198 | 8 |
| Diethyl phthalate | 79 | 1.20 | 222 | 4 |
| Nonanediol-1,3-diacetate | 33 | 1.17 | 244 | 8 |
| Nonanolide-1,4 | 92 | 0.87 | 156 | 5 |
| i-Nonyl acetate | 50 | 0.83 | 186 | 4 |
| i-Nonyl formate | 19 | 1.49 | 172 | 8 |
| Phenylethyl phenyl acetate | 0 | 1.22 | 241 | 7 |

Class 6 - Alcohols & Ethers

| Cinnamic alcohol | - | 1.28 | 134 | 9 |
|---|---|---|---|---|
| Dimyrcetol | 16 | 1.22 | 156 | 9 |
| 1,3,4,6,7,8-Hexahydro-4,6,6,7,8,8-hexamethyl cyclopenta-ɣ-2-benzopyran (Galaxolide) | 100 | - | 240 | 5 |
| Hydroxymethyl isopropyl cyclopentane | 60 | 1.23 | 142 | 8 |
| 3a-Methyl-dodecahydro-6,6,9a-trimethylnaphtho-2(2,1-b)furan | 58 | 1.30 | 230 | 5 |
| Tetrahydromuguol | 24 | 1.23 | 158 | 8 |

Examples of perfume ingredients that are not bleach-stable, (which accordingly are not likely to contribute substantially to the deodorant properties of the perfume when formulated in the presence of bleach materials) are as follows:

EP 0 404 470 B1

| | LIC value | RVR value | $\underline{m}$ | BST panel score |
|---|---|---|---|---|
| Benzoin Siam resinoids | 87 | – | – | 14 |
| Benzyl salicylate | 0 | 1.58 | 228 | 17 |
| Bergamot AB 37 | 58 | 0.97 | 175 | 10 |
| p-t-Butyl cyclohexyl acetate | 54 | 0.98 | 198 | 10 |
| p-t-Butyl-$\alpha$-methyl hydrocinnamic aldehyde (Lilial) | 74 | – | 204 | 13 |
| Coumarin | 58 | 1.22 | 146 | 10 |
| Ethyl vanillin | 100 | 1.43 | 152 | 12 |
| Geranium oil Bourbon | 26 | 1.29 | 154 | 10 |
| LRG 201 (a well known resorcylic acid ester) | 100 | 1.21 | 196 | 11 |
| Mousse de chene Yugo | 98 | 1.29 | 182 | 11 |
| $\beta$-Naphthyl methyl ether | 100 | – | 158 | 11 |
| Opoponax resinoid | 96 | 1.33 | 150 | 11 |
| Patchouli oil | 76 | 1.25 | 140 | 11 |
| Petitgrain oil | 34 | 1.27 | 175 | 11 |
| Phenylethyl alcohol | 22 | 1.24 | 122 | 10 |
| Pimento leaf oil | 100 | – | 165 | 12 |
| Pomeransol AB 314 | 100 | – | – | 11 |

Of the components and ingredients listed herein, those that are preferred for their relatively milder odour intensity are (a) the ingredients included in the examples given below, and (b)

Bergamot AB,
Hexyl salicylate,
Rose AB 380,
Diethyl phthalate,
Nonanediol 1,3-diacetate,
Isononyl acetate,
Cinnamic alcohol,
Benzoin Siam resinoid,
Benzyl salicylate,
p-t-butyl-alpha-methyl-hydrocinnamic aldehyde,
Opoponax resinoid.

A deodorant perfume should contain at least five different components, and from preferably at least four of the classes, preferaby five or six. It is however possible, and indeed is usually advantageous, to employ more than five different components when formulating the perfume. Ideally, most if not all of the perfume is formulated from deodorant perfume components.

Components present in the deodorant perfume for purposes other than obtaining a deodorant effect, for example an adjunct like an anti-oxidant, may be excluded from the operation of the preceding instructions to the extent that the component is required for that other purpose. The levels at which adjuncts are conventionally present in perfumes or in products to which perfumes are added is well-established for conventional ma-

8

terials and readily determinable for new materials so that the application of the above exclusion presents no difficulty.

Odour Intensity Index Method

The samples are assessed by a panel of a suitable number, e.g. about 34, of assessors who have been trained to score the intensity of a sample using the magnitude estimation technique. This is a ratio scaling method in which the relative intensity of each sample is scored in ratio to the intensities of a range of odour standards (here, benzyl acetate diluted in dipropylene glycol at various concentrations.

1.5g (+/- 0.1g) of perfume, or 1.5g (+/-0.1g) of benzyl acetate either neat or as a dilution in dipropylene glycol, is placed into 7ml white soda S.N.B. screw neck vials with 19mm diameter necks. The samples are each coded and presented to the panel in a random order at least twice. A total of at least 64 assessments (or enough to reach statistical significance) is made for each sample by at least 16 panellists on each day over two days. Assessments are made in environmentally controlled assessment rooms using coloured lighting to ensure that panellists are not influenced by any slight colour differences between the samples.

Individual assessments are normalised and averaged to give a consensus intensity rating for each sample. The perceived intensities are expressed in arbitrary units and are derived from consensus magnitude estimates which are indicative of the ratio of perceived intensities, as follows:-

Each panellist was required to assess the intensity of a control sample (10% benzyl acetate solution in dipropylene glycol) in addition to each test fragrance and the reference samples. The intensity value (magnitude estimate) of the control sample was then used to normalise all the other assessments for each panellist as follows:-

$$\text{Normalised Intensity} = \frac{\text{Intensity of Unknown}}{\text{Intensity of control}} \times 100.$$

or:

$$(I_N)_j = \frac{(i_K)_j}{(i_c)_j} \times 100$$

The normalised values for a sample were combined across all panellists to give a consensus value for the whole panel (the arithmetic mean).

$$\text{Odour intensity index} = \Sigma \frac{(\text{normalised panellist ratings})}{\text{number of panellists.}}$$

or:

$$I_K = \sum_{1}^{J} \frac{(I_N)_j}{J}$$

KEY

$I_K$ =             odour intensity index for sample k for the whole panel.
$(i_K)_j$ =       odour intensity of sample (magnitude estimate) as reported by j'th panellist.
$(I_N)_j$ =      single panellist's normalised datum.
$n$ =             number of samples.
$J$ =             number of panellists
$K$ =            sample number.
$(i_c)_j$ =        odour intensity of control (magnitude estimate) as reported by j'th panellist.

The invention is further illustrated by the following non-limitative examples.

Example 1

A fragrance composition suitable for use in this invention is formulated as follows:-

| % | Component |
|---|---|
| 2.0 | Cedar wood oil (Virginian) |
| 2.0 | Cinnamic Alcohol |
| 13.0 | Diethyl Phthalate |
| 5.0 | Galaxolide DEP (50:50 mixture with diethyl phthalate) |
| 4.0 | Geranyl Phenylacetate |
| 1.0 | Guaiacwood oil (rectified) |
| 4.0 | Linalyl Benzoate |
| 6.0 | Moss Base AB 7004 (*) |
| 3.0 | Phenylethyl Phenylacetate |
| 20.0 | Rose Base AB 7003 (*) |
| 40.0 | Traseolide (Quest) |
| 100.0 | |

(*) (available from Quest International)

The odour type of this formulation is mildly floral, mossy, rose, and musk. The composition is of low odour intensity and is suitable for incorporation into (inter alia) fabric - washing detergent compositions for example at a rate of incorporation of about 0.2% by weight of the detergent formulation.

Example 2

A further fragrance composition according to the invention is as follows:-

| % | Component |
|---|---|
| 8.0 | Benzyl Alcohol |
| 7.5 | Benzyl Salicylate |
| 2.0 | Cedar wood oil (Virginian) |
| 20.0 | Galaxolide DEP |
| 1.0 | Grisalva (10% solution in dipropylene glycol) (IFF) |
| 5.0 | Hercolyn D (Hercules) |
| 3.0 | Isobutyl Benzoate |
| 2.0 | Isobutyl Cinnamate |
| 1.0 | Linalyl Cinnamate |
| 5.0 | Moss Base AB 7004 (*) |
| 20.0 | Muguet Base AB 7001 (*) |
| 5.0 | Tonalid (7-acetyl-1,1,3,4,4,6-hexamethyl-tetrahydronaphthalene) (Polak's Frutal Works) |
| 20.0 | Traseolide (*) |
| 100.0 | |

The odour type of this formulation is mildly woody, mossy, muguet and musk. "Hercolyn" and "Tonalid" are Registered Trade Marks.

Example 3

A further and highly preferred composition for extremely low odour intensity is as follows:

| % | Component |
|---|---|
| 5.0 | Benzyl Alcohol |
| 4.0 | Benzyl Cinnamate |
| 20.0 | Benzyl Salicylate |
| 1.0 | Cinnamyl Cinnamate |
| 5.0 | Diethyl Phthalate |
| 8.0 | Galaxolide DEP |
| 20.0 | Jasmin AB 7002 (*) |
| 5.0 | Linalyl Cinnamate |
| 2.0 | Sandalone AC 802 (*) |
| 30.0 | Traseolide (*) |
| 100.0 | |

The odour type of this formulation is mildly sweet, floral and musk.

Example 4

A further example of a perfumery composition in accordance with the invention is as follows:-

| % | Component |
|---|---|
| 18.0 | Benzyl Salicylate |
| 2.0 | Diethyl Phthalate |
| 5.0 | Ethylene Brassylate |
| 10.0 | Galaxolide DEP |
| 20.0 | Muguet AB 7001 (*) |
| 2.0 | Phenylethyl Salicylate |
| 2.0 | Sandalone AC 802 (*) |
| 3.0 | Sandela (Givaudan) |
| 38.0 | Traseolide (*) |
| 100.0 | |

The odour type of this formulation is mildly muguet and musk.

Example 5

A further example of a composition according to the invention is as follows:-

| % | Component |
|---|---|
| 5.0 | Benzyl Alcohol |
| 8.0 | Benzyl Benzoate |
| 5.0 | Benzyl Cinnamate |
| 5.0 | Carnation AB 7005 (*) |
| 2.0 | Copaiba Balsam |
| 10.0 | Galaxolide DEP |
| 15.0 | Hexyl Salicylate |
| 15.0 | Jasmin Base AB 7002 (*) |
| 35.0 | Traseolide (*) |
| | |
| 100.0 | |

The odour type of this formulation is mildly spicy, jasmin and musk.

Odour Intensity Indices

The odour intensity indices of the above-cited examples are as follows:

| Composition | Odour Intensity Index |
|---|---|
| Example 1 | 87 |
| Example 2 | 85 |
| Example 3 | 72 |
| Example 4 | 79 |
| Example 5 | 75 |

For comparison and calibration, standard preparations of benzyl acetate have odour intensity indices as follows:-

| Reference Standard | Perceived Odour Index |
|---|---|
| 1% Benzyl Acetate | 54 |
| 5% Benzyl Acetate | 89 |
| 10% Benzyl Acetate | 102 |
| 20% Benzyl Acetate | 108 |
| 50% Benzyl Acetate | 120 |
| Neat Benzyl Acetate | 132 |

(The standard samples of benzyl acetate are presented as dilutions in dipropylene glycol.)

## Claims

1. A composition of fragrance materials having an odour intensity less than that of a 10% solution of benzyl acetate in dipropylene glycol, and a Malodour Reduction Value of at least 0.25 or an Odour Reduction Value of at least 0.25.

2. Use of a composition as defined in claim 1 as a fragrance composition in a detergent powder, a detergent liquid, a soap or detergent bar or paste, a fabric-conditioning composition in liquid or solid form, or a personal body deodorant composition.

3. A detergent composition suitable for fabric-washing, comprising a detergent surfactant and detergent adjuncts, including a perfume composition, characterised by a content of perfume ingredients in the perfume composition containing (a) optionally at least 0.03% of a musk, based on the weight of the detergent composition, and (b) at least 0.07% of other perfume ingredients, wherein the fragrance components (a) (if present) and (b) together constitute a composition as defined in claim 1.

4. A composition according to claim 1 or 3, wherein the fragrance materials or perfume ingredients comprise at least 30% by weight of a musk.

5. A composition according to claim 1 or 3, wherein the fragrance materials or perfume ingredients comprise at least 35% by weight of a musk.

6. A composition according to claim 3, in which the fragrance of the perfume ingredients in the detergent composition is so low as to be imperceptible in use.

7. A composition according to claim 3, wherein the perfume composition (considered apart from constituents individually making up less than 0.5% of the composition) contains at least five different components in the following classes;
   class 1 - phenolic substances; class 2 - essential oils, extracts, resins and synthetic
      oils; class 3 - aldehydes and ketones; class 4 - nitrogen-containing compounds and polycyclic
      compounds; class 5 - esters; class 6 - alcohols and ethers;
   and at least four of the classes are represented in the composition;
   (provided that where an individual component is allocatable to more than one of such classes it is allocated to the lowest-numbered class).

8. A composition according to any of claims 1 or 3 to 7, wherein the perfume ingredients or fragrance materials comprise ingredients selected from:
   alpha-iso-methyl ionone, benzoin Siam resinoid, Bergamot AB 430,
   isoamyl salicylate, carvacrol, clove leaf oil, ethyl salicylate, iso-eugenol, hexyl salicylate, thyme oil red,
   Bergamot AB 430, Geranium AB 76, Rose AB 380, Rose AB 409,
   7-acetyl-1,1,3,4,4,6-hexamethyl-tetrahydronaphthalene, p-t-amyl cyclohexanone, 2-n-heptyl cyclopentanone, alpha-iso-methyl ionone, beta-methyl naphthyl ketone,
   iso-butyl quinoline, methyl anthranilate,
   o-t-butyl cyclohexyl acetate, diethyl phthalate, nonanediol-1,3-diacetate, nonanolide-1,4, i-nonyl acetate,
   i-nonyl formate, phenylethyl phenyl-acetate,
   cinnamic alcohol, dimyrcetol, 1,3,4,6,7,8-hexahydro-4,6,6,7,8,8-hexamethyl-cyclopenta-gamma-2-benzopyran (galaxolide), hydroxymethyl isopropyl cyclopentane, 3a-methyl-dodecahydro-6,6,9a-trimethylnaphtho-2(2,1-b)-furan, tetrahydromuguol.

9. A composition according to any of claims 1 or 3 to 7, wherein the perfume ingredients or fragrance materials comprise negligible amounts of the following (bleach-unstable) ingredients:
   benzoin Siam resinoids, benzyl salicylate, Bergamot AB 37, p-t-butyl cyclohexyl acetate, p-t-butyl alphamethyl hydrocinnamic aldehyde (lilial), coumarin, ethyl vanillin, geranium oil Bourbon, resorcylic acid ester LRG 201, mousse de chene Yugo, beta-naphthyl methyl ether, opoponax resinoid, patchouli oil, petitgrain oil, phenyl ethyl alcohol, pimento leaf oil, pomeransol AB 314.

10. A composition according to any of claims 1 or 3 to 7, wherein the perfume ingredients or fragrance materials comprise ingredients selected from:

14

Bergamot base AB, hexyl salicylate, Rose base AB 380, diethyl phthalate, nonane-diol 1,3-diacetate, isononyl acetate, cinnamic alcolhol, benzoin Siam resinoid, benzyl salicylate, p-t-butyl alpha-methyl hydrocinnamic aldehyde (lilial), opoponax resinoid.

11. A composition according to any of claims 1 or 3 to 7, wherein the perfume ingredients or fragrance materials comprise ingredients selected from:
cedar wood oil (virginian), cinnamic alcohol, diethyl phthalate, galaxolide, geranyl phenyl-acetate, guaiacwood oil (rectified), linalyl benzoate, Moss base AB 7004, phenylethyl phenyl-acetate, Rose base AB 7003.

12. A composition according to any of claims 1 or 3 to 7, wherein the perfume ingredients or fragrance materials comprise ingredients selected from:
benzyl alcohol, benzyl salicylate, cedarwood oil (virginian), galaxolide, diethyl phthalate, grisalva, Hercolyn D, isobutyl benzoate, isobutyl cinnamate, linalyl cinnamate, Moss base AB 7004, Muguet base AB 7001, 7-acetyl-1,1,3,4,4,6-hexamethyl tetrahydronaphthalene (Tonalid), traseolide.

13. A composition according to any of claims 1 or 3 to 7, wherein the perfume ingredients or fragrance materials comprise ingredients selected from:
benzyl alcohol, benzyl cinnamate, benzyl salicylate, cinnamyl cinnamate, diethyl phthalate, galaxolide, diethyl phthalate, Jasmin base AB 7002, linalyl cinnamate, Sandalone AC 802, Traseolide.

14. A composition according to any of claims 1 or 3 to 7, wherein the perfume ingredients or fragrance materials comprise ingredients selected from:
benzyl salicylate, diethyl phthalate, ethylene brassylate, galaxolide, Muguet base AB 7001, phenylethyl salicylate, Sandalone AC 802, Sandela (ex Givaudan), traseolide.

15. A composition according to any of claims 1 or 3 to 7, wherein the perfume ingredients or fragrance materials comprise ingredients selected from:
benzyl alcohol, benzyl benzoate, benzyl cinnamate, Carnation base AB 7005, copaiba balsam, galaxolide, diethyl phthalate, hexyl salicylate, Jasmin base AB 7002, traseolide.

16. A composition according to any of claims 1 or 3 to 7, wherein a majority of the perfume ingredients or fragrance materials are selected from those listed in claims 8 to 15.

17. A composition according to any of claims 1 or 3 to 7, wherein the perfume ingredients or fragrance materials consist essentially of ingredients selected from those listed in claims 8 to 15.

18. A composition according to any of claims 1 or 3 to 7, wherein a majority of the perfume ingredients or fragrance materials are selected from those listed in claims 8 to 15, excluding those listed in claim 9.

19. A composition according to any of claims 1 or 3 to 7, wherein the perfume ingredients or fragrance materials consist essentially of ingredients selected from those listed in claims 8 to 15, excluding those listed in claim 9.

20. A composition according to any of claims 1 or 3 to 7, wherein a majority of the perfume ingredients or fragrance materials are selected from those listed in claims 10 to 15.

21. A composition according to any of claims 1 or 3 to 7, wherein the perfume ingredients or fragrance materials consist essentially of ingredients selected from those listed in claims 10 to 15.

22. A composition according to any of claims 1 or 3 to 7, wherein the perfume ingredients or fragrance materials are substantially bleach-stable.

## Patentansprüche

1. Duftstoffzusammensetzung mit einer Geruchsintensität, die geringer als die einer 10%igen Lösung von Essigsäure benzylester in Dipropylenglycol ist, und einem Gestankreduktionswert von mindestens 0.25 oder einem Geruchtsreduktionswert von mindestens 0.25.

2. Verwendung einer Zusammensetzung nach Anspruch 1 als Duftzusammensetzung in einem Reinigungspulver, einer Reinigungsflüssigkeit, einer Seife oder einem Reinigungsriegel oder einer -paste, einer Gewebe-Weichspül-Zusammensetzung in flüssiger oder fester Form oder einer persönlichen Körperdeodorant-Zusammensetzung.

3. Reinigungszusammensetzung, die zur Gewebereinigung geeignet ist, welche ein oberflächenaktives Reinigungsmittel und Reinigungszusätze enthält, einschließlich einer Parfümzusammensetzung, gekennzeichnet durch einen Gehalt an Parfüminhaltsstoffen in der Parfümzusammensetzung, enthaltend (a) gegebenenfalls mindestens 0,03% eines Moschusaromas, bezogen auf das Gewicht der Reinigungszusammensetzung und (b) mindestens 0,07% anderer Parfüminhaltsstoffe, wobei die Duftbestandteile (a) (sofern vorhanden) und (b) zusammen eine Zusammensetzung nach Anspruch 1 bilden.

4. Zusammensetzung nach Anspruch 1 oder 3, wobei die Duftstoffe oder Parfüminhaltsstoffe mindestens 30 Gew.-% eines Moschusaromas enthalten.

5. Zusammensetzung nach Anspruch 1 oder 3, wobei die Duftstoffe oder Parfüminhaltsstoffe mindestens 35 Gew.-% eines Moschusaromas enthalten.

6. Zusammensetzung nach Anspruch 3, wobei der Duft der Parfüminhaltsstoffe in der Reinigungszusammensetzung so gering ist, daß er bei der Verwendung nicht wahrgenommen wird.

7. Zusammensetzung nach Anspruch 3, wobei die Parfümzusammensetzung (abgesehen von Bestandteilen, die individuell zusammen weniger als 0,5% der Zusammensetzung bilden) mindestens fünf verschiedene Bestandteile aus den folgenden Klassen enthält;
Klasse 1 - Phenolische Substanzen; Klasse 2 - Essentielle Öle, Extrakte, Harze und synthetische Öle; Klasse 3 - Aldehyde und Ketone; Klasse 4 - Stickstoffhaltige Verbindungen und polycyclische Verbindungen; Klasse 5 - Ester; Klasse 6 - Alkohole und Ether;
und mindestens vier der Klassen in der Zusammensetzung vertreten sind;
(unter der Voraussetzung, daß wenn ein individueller Bestandteil zu mehr als einer dieser Klassen zuordbar ist, er der Klasse mit der niedrigsten Zahl zugeordnet wird).

8. Zusammensetzung nach einem der Ansprüche 1 oder 3 bis 7, wobei die Parfüminhaltsstoffe oder Duftstoffe Inhaltsstoffe enthalten, die ausgewählt sind aus:
$\alpha$-Isomethylionon, Benzoin-Siam-Resinoid, Bergamot AB 430, Salicylsäureisoamylester, Carvacrol, Gewürznelkenblattöl, Salicylsäureethylester, Iso-Eugenol, Salicylsäurehexylester, rotem Thymianöl,
Bergamot AB 430, Geranium AB 76, Rose AB 380, Rose AB 409,
7-Acetyl-1,1,3,4,4,6-tetrahydronaphthalin, p-t-Amyl-cy clohexanon, 2-n-Heptyl-cyclopentanon, Alpha-iso-Methyl ionon, Beta-Methyl-naphthyl-keton,
Iso-butyl-chinolin, Anthranilsäuremethylester,
Essigsäure-o-t-butyl-cyclohexylester, Phthalsäurediethylester, Nonandiol-1,3-diacetat, Nonanolid-1,4, Essigsäure-i-nonylester, Ameisensäure-i-nonylester, Phenylessigsäurephenylethylester,
Zimtalkohol, Dimyrectol, 1,3,4,6,7,8-Hexahydro-4,6,6,7,8,8-hexamethyl-cyclopenta-gamma-2-benzopyran (Galaxolid), Hydroxymethyl-isopropyl-cyclopentan, 3a-Methyl-dodecahydro-6,6,9a-trimethyl-naphto-2(2,1-b)-furan, Tetrahydromuguol.

9. Zusammensetzung nach einem der Ansprüche 1 oder 3 bis 7, wobei die Parfüminhaltsstoffe oder Duftstoffe geringfügige Mengen der folgenden (bleichmittellabilen) Inhaltsstoffe enthalten:
Benzoin-Siam-Resinoide, Salicylsäurebenzylester, Bergamot AB 37, Essigsäure-p-t-butyl-cyclohexylester, p-t-Butyl-alpha-methyl-hydrozimtaldehyd (Lilial), Coumarin, Ethyl-vanillin, Bourbon-Geranienöl, Resorcylsäureester LRG 201, Mousse de chene Yugo, Beta-Naphthyl-methyl-ether, Opoponax-Resinoid, Patchouli-Öl, Petigrain-Öl, phenyl-ethyl-Alkohol, Pimentblattöl, Pomeransol AB 314.

10. Zusammensetzung nach einem der Ansprüche 1 oder 3 bis 7, wobei die Parfüminhaltsstoffe oder Duftstoffe Inhaltsstoffe enthalten, die ausgewählt sind aus:
Bergamot-Basis AB, Salicylsäurehexylester, Rose-Basis AB 380, Phthalsäurediethylester, Nonan-diol-1,3-diacetat, Essigsäureisononylester, Zimtalkohol, Benzoin-Siam-Resinoid, Salicylsäurebenzylester, p-t-Butyl-alpha-methyl-hydrozimtaldehyd (Lilial), Opoponax-Resinoid.

11. Zusammensetzung nach einem der Ansprüche 1 oder 3 bis 7, wobei die Parfüminhaltsstoffe oder Duftstoffe Inhaltsstoffe enthalten, die ausgewählt sind aus:
Zedernholzöl (Virginisch) Zimtalkohol, Phthalsäurediethylester, Galaxolid, Phenylessigsäuregeranylester, Guaiacholzöl (rektifiziert), Benzoesäurelinalylester, Moos-Basis-AB 7004, Phenylessigsäurephenylethylester, Rose-Basis AB 7003.

12. Zusammensetzung nach einem der Ansprüche 1 oder 3 bis 7, wobei die Parfüminhaltsstoffe oder Duftstoffe Inhaltsstoffe enthalten, die ausgewählt sind aus:
Benzylalkohol, Salicylsäurebenzylester, Zedernholzöl (Virginisch) Galaxolid, Phthalsäurediethylester, Grisalva, Hercolyn D, Benzoesäureisobutylester, Zimtsäureisobutylester, Zimtsäurelinalylester, Moos-Basis AB 7004, Muguet-Basis AB 7001, 7-Acetyl-1,1,3,4,4,6-hexamethyltetrahydronaphthalin (Tonalid), Traseolid.

13. Zusammensetzung nach einem der Ansprüche 1 oder 3 bis 7, wobei die Parfüminhaltsstoffe oder Duftstoffe Inhaltsstoffe enthalten, die ausgewählt sind aus:
Benzylalkohol, Zimtsäurebenzytester, Salicylsäurebenzylester, Zimtsäurecinnamylester, Phthalsäurediethylester, Galaxolid, Phthalsäurediethylester, Jasmin-Basis AB 7002, Zimtsäurelinalylester, Sandalon AC 802, Traseolid.

14. Zusammensetzung nach einem der Ansprüche 1 oder 3 bis 7, wobei die Parfüminhaltsstoffe oder Duftstoffe Inhaltsstoffe enthalten, die ausgewählt sind aus:
Salicylsäurebenzylester, Phthalsäurediethylester, Brassylsäureethylenester, Galaxolid, Muguet-Basis AB 7001, Salicylsäurephenylethylester, Sandalon AC 802, Sandela (ex Givaudan), Traseolid.

15. Zusammensetzung nach einem der Ansprüche 1 oder 3 bis 7, wobei die Parfüminhaltsstoffe oder Duftstoffe Inhaltsstoffe enthalten, die ausgewählt sind aus:
Benzylalkohol, Benzoesäurebenzylester, Zimtsäurebenzylester, Gartennelken-Basis AB 7005, Copaiba-Balsam, Galaxolid, Phthalsäurediethylester, Salicylsäurehexylester, Jasmin-Basis AB 7002, Traseolid.

16. Zusammensetzung nach einem der Ansprüche 1 oder 3 bis 7, wobei der Hauptteil der Parfüminhaltsstoffe oder Duftstoffe aus den in den Ansprüchen 8 bis 15 angegebenen ausgewählt ist.

17. Zusammensetzung nach einem der Ansprüche 1 oder 3 bis 7, wobei die Parfüminhaltsstoffe oder Duftstoffe im wesentlichen aus Inhaltsstoffen bestehen, die aus den in den Ansprüchen 8 bis 15 angegebenen ausgewählt sind.

18. Zusammensetzung nach einem der Ansprüche 1 oder 3 bis 7, wobei der Hauptteil der Parfüminhaltsstoffe oder Duftstoffe aus den in den Ansprüchen 8 bis 15 angegebenen ausgewählt ist, mit Ausnahme der in Anspruch 9 angegebenen.

19. Zusammensetzung nach einem der Ansprüche 1 oder 3 bis 7, wobei die Parfüminhaltsstoffe oder Duftstoffe im wesentlichen aus Inhaltsstoffen bestehen, die aus den in den Ansprüchen 8 bis 15 angegebenen ausgewählt sind, mit Ausnahme der in Anspruch 9 angegebenen.

20. Zusammensetzung nach einem der Ansprüche 1 oder 3 bis 7, wobei der Hauptteil der Parfüminhaltsstoffe oder Duftstoffe aus den in den Ansprüchen 10 bis 15 angegebenen ausgewählt ist.

21. Zusammensetzung nach einem der Ansprüche 1 oder 3 bis 7, wobei die Parfüminhaltsstoffe oder Duftstoffe im wesentlichen aus Inhaltsstoffen bestehen, die aus den in den Ansprüchen 10 bis 15 angegebenen ausgewählt sind.

22. Zusammensetzung nach einem der Ansprüche 1 oder 3 bis 7, wobei die Parfüminhaltsstoffe oder Duftstoffe im wesentlichen bleichmittelstabil sind.

## Revendications

1. Composition de parfum dont l'intensité d'odeur est inférieure à celle d'une solution à 10% d'acétate de benzyle dans le dipropylène-glycol et dont l'indice de réduction de mauvaise odeur est d'au moins 0,25 ou bien l'indice de réduction d'odeur est d'au moins 0,25.

2. Utilisation d'une composition selon la revendication 1, en qualité d'une composition de parfum dans une poudre détergente, un liquide détergent, un pain ou pâte de savon ou de détergent, une composition de conditionnement de textile sous forme liquide ou solide ou une composition déodorante de toilette.

3. Composition détergente convenant pour le lavage des textiles et qui comprend un tensioactif détergent et des additifs aux détergents notamment une composition de parfum, caractérisée en ce que la teneur en ingrédients de parfum dans la composition de parfum contient (a) facultativement au moins 0,03% d'un musc, par rapport au poids de la composition détergente et (b) au moins 0,07% d'autres ingrédients de parfum, les composants de fragrance (a) (si présents) et (b) constituant conjointement une composition telle que définie dans la revendication 1.

4. Composition selon la revendication 1 ou 3, dans laquelle les matières de fragrance ou les ingrédients de parfum comprennent au moins 30% en poids d'un musc.

5. Composition selon la revendication 1 ou 3, dans laquelle les matières de fragrance ou les ingrédients de parfum comprennent au moins 35% en poids d'un musc.

6. Composition selon la revendication 3, dans laquelle la fragrance des ingrédients de parfum dans la composition détergente est tellement basse qu'elle est imperceptible en utilisation.

7. Composition selon la revendication 3, dans laquelle la composition de parfum (considérée séparément des constituants qui totalisent individuellement moins de 0,5% de la composition) contient au moins 5 composants différents dans les catégories suivantes :
catégorie 1 : substances phénoliques ;
catégorie 2 : huiles essentielles, extraits, résines et huiles synthétiques ;
catégorie 3 : aldéhydes et cétones ;
catégorie 4 : composés azotés et composés polycycliques ;
catégorie 5 : esters ;
catégorie 6 : alcools et éthers ;
et au moins quatre des catégories sont représentées dans la composition ;
(à la condition que si un composant individuel peut être incorporé dans plus d'une de ces catégories, on l'attribue à la catégorie ayant le numéro le plus bas).

8. Composition selon l'une quelconque des revendications 1 ou 3 à 7 dans laquelle les ingrédients de parfum ou les matières de fragrance comprennent des ingrédients choisis parmi les suivantes :
alpha-isométhylionone, résinoide benzoinique de Siam, Bergamot AB 430,
salicylate d'isoamyle, carvacrol, essence de clou de girofle, salicylate d'éthyle, iso-eugenol, salicylate d'hexyle, rouge d'essence de thym,
Bergamot AB 430, Geranium AB 76, Rose AB 380, Rose AB 409,
7-acétyl-1,1,3,4,4,6-hexaméthyltetrahydronaphtalène, p,t-amylcyclohexanone, 2-n-heptyl-cyclopentanone, alpha-isométhylionone, bêta-méthylnaphtylcétone,
isobutylquinoléine, anthranilate de méthyle,
acétate d'o-t-butyl-cyclohexyle, phtalate de diéthyle, 1,3-diacétate de nonanediol, nonalide-1,4, acétate d'isononyle, formiate d'iso-nonyle, acétate de phényléthylphényle,
alcool cinnamique, dimyrcétol, 1,3,4,6,7,8-hexahydro-4,6,6, 7,8,8-hexaméthyl-cyclopenta-gamma-2-benzopyrane (galaxolide), hydroxyméthyl-isopropyl-cyclopentane, 3a-méthyl-dodecahyddro-6,6,9a-triméthyl-naphto-2(2,1-b)-furane, tetrahydromuguol.

9. Composition selon l'une quelconque des revendications 1 ou 3 à 7, dans laquelle les ingrédients de parfum ou les matières de fragrance comprennent des quantités négligeables des ingrédients suivants instables au blanchiment :
résinoïdes benzoïniques de Siam, salicylate de benzyle, Bergamot AB 37, acétate de p-t-butylcyclohexyle, aldéhyde p-t-butylalphaméthylhydrocinnamique (lilial), coumarine, éthyl-vanilline, essence de géranium Bourbon, ester d'acide resorcylique LRG 201, mousse de chêne Yugo, éther bêta-naphtylméthylique, résinoide opoponax, essence de patchouli, essence de petitgrain, alcool phényléthylique, essence de feuille de piment, pomeransol AB 314.

10. Composition selon l'une quelconque des revendications 1 ou 3 à 7, dans laquelle les ingrédients de parfum ou matière de fragrance comprennent les ingrédients choisis parmi les suivants :

base de Bergamot AB, salicylate d'hexyle, base de Rose AB 380, phtalate de diéthyle, 1,3-diacétate de nonane-diol, acétate d'isononyle, alcool cinnamique, résinoïde benzoïnique de Siam, salicylate de benzyle, aldéhyde p-t-butyl-alpha-méthyl-hydrocinnamique (lilial), résinoide opoponax.

11. Composition selon l'une quelconque des revendications 1 ou 3 à 7, dans laquelle les ingrédients de parfum ou les matières de fragrance comprennent les ingrédients choisis parmi les suivants : essence de bois de cèdre de Virginie, alcool cinnamique, phtalate de diéthyle, galaxolide, acétate phénylique de géranyle, essence de bois de guaiac (redressée), benzoate de linalyle, base de mousse AB 7004, acétate de phényléthylphényle, base de rose AB 7003.

12. Composition selon l'une quelconque des revendications 1 ou 3 à 7, dans laquelle les ingrédients de parfum ou les matières de fragrance comprennent les ingrédients choisis parmi les suivants :
alcool benzylique, salicylate de benzyle, essence de bois de cèdre de Virginie, galaxolide, phtalate de diéthyle, grisalva, Hercolyn D, benzoate d'isobutyle, cinnamate d' isobutyle, cinnamate de linalyle, base de mousse AB 7004, base de muguet AB 7001, 7-acétyl-1,1,3,4,4,6-hexaméthyltetrahydronaphtalène (Tonalid), Traseolide.

13. Composition selon l'une quelconque des revendications 1 ou 3 à 7, dans laquelle les ingrédients de parfum ou les matières de fragrance comprennent des ingrédients choisis parmi les suivants :
alcool benzylique, cinnamate de benzyle, salicylate de benzyle, cinnamate de cinnamyle, phtalate de diéthyle, galaxolide, phtalate de diéthyle, base de Jasmin AB 7002, cinnamate de linalyle, Sandalone AC 802, Traseolide.

14. Composition selon l'une quelconque des revendications 1 ou 3 à 7, dans laquelle les ingrédients de parfum ou les matières de fragrance comprennent des ingrédients choisis parmi les suivants :
salicylate de benzyle, phtalate de diéthyle, brassylate d'éthylène, galaxolide, base de muguet AB 7001, salicylate de phényléthyle, Sandalone AC 802, Sandela (Givaudan), Traseolide.

15. Composition selon l'une quelconque des revendications 1 ou 3 à 7, dans laquelle les ingrédients de parfum ou les matières de fragrance comprennent des ingrédients choisis parmi les suivants :
alcool benzylique, benzoate de benzyle, cinnamate de benzyle, base d'oeillet AB 7005, baume de copaïba, galaxolide, phtalate de diéthyle, salicylate d'hexyle, base de Jasmin AB 7002, Traséolide.

16. Composition selon l'une quelconque des revendications 1 ou 3 à 7, dans laquelle la majorité des ingrédients de parfum ou des matières de fragrance sont choisis parmi ceux énumérés dans les revendications 8 à 15.

17. Composition selon l'une quelconque des revendications 1 ou 3 à 7, dans laquelle les ingrédients de parfum ou les matières de fragrance consistent essentiellement en ceux énumérés dans les revendications 8 à 15.

18. Composition selon l'une quelconque des revendications 1 ou 3 à 7, dans laquelle la majorité des ingrédients de parfum ou de matières de fragrance sont choisis parmi ceux énumérés dans les revendications 8 à 15, à l'exclusion de ceux énumérés dans la revendication 9.

19. Composition selon l'une quelconque des revendications 1 ou 3 à 7, dans laquelle les ingrédients de parfum ou les matières de fragrance sont essentiellement des ingrédients choisis parmi ceux énumérés dans les revendications 8 à 15, à l'exclusion de ceux énumérés dans la revendication 9.

20. Composition selon l'une quelconque des revendications 1 ou 3 à 7, dans laquelle la majorité des ingrédients de parfum ou de matière de fragrance sont choisis parmi ceux des revendications 10 à 15.

21. Composition selon l'une quelconque des revendications 1 ou 3 à 7, dans laquelle les ingrédients de parfum ou les matières de fragrance sont essentiellement ceux énumérés dans les revendications 10 à 15.

22. Composition selon l'une quelconque des revendications 1 ou 3 à 7, dans laquelle les ingrédients de parfum ou les matières de fragrance sont sensiblement stables au blanchiment.